Europäisches Patentamt

European Patent Office

Office européen des brevets

(19)

(11) Veröffentlichungsnummer: **0 289 846**
**A1**

(12) **EUROPÄISCHE PATENTANMELDUNG**

(21) Anmeldenummer: 88106174.1

(22) Anmeldetag: 19.04.88

(51) Int. Cl.⁴: **C12N 15/00 , C12P 13/22 ,**
**C12N 9/10 , //(C12N15/00,**
**C12R1:38),(C12N15/00,**
**C12R1:19),(C12N15/00,**
**C12R1:43),(C12N15/00,**
**C12R1:39)**

(30) Priorität: 24.04.87 DE 3713755

(43) Veröffentlichungstag der Anmeldung:
09.11.88 Patentblatt 88/45

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Marquardt, Rüdiger, Dr.
Günthersburgallee 69
D-6000 Frankfurt am Main 60(DE)
Erfinder: Then, Johann, Dr.
Sulzbacher Weg 2
D-6230 Hofheim am Taunus(DE)
Erfinder: Bräu, Barbara, Dr.
Johannesallee 8
D-6230 Frankfurt am Main 80(DE)
Erfinder: Wöhner, Gerhard, Dr.
Flörsheimer Strasse 27
D-6093 Flörsheim am Main(DE)
Erfinder: Präve, Paul, Prof. Dr.
Kronberger Weg 7
D-6231 Sulzbach (Taunus)(DE)

(54) Herstellung von L-Phenylalanin mit Hilfe rekombinanter Bakterien.

(57) Fumarsäure, Ammoniumionen und Phenylpyruvat können von genetisch manipulierten Mikroorganismen, die ein Aspartase-Gen sowie ein Aminotransferase-Gen auf einem replizierenden extra-chromosomalen Element oder getrennt auf zwei kompatiblen Plasmiden enthalten, die in dem betreffenden Mikroorganismus exprimiert werden, in hohen Geschwindigkeiten zu L-Phenylalanin transformiert werden.

EP 0 289 846 A1

## Herstellung von L-Phenylalanin mit Hilfe rekombinanter Bakterien

Der letzte Schritt der de novo-Synthese von L-Phenylalanin besteht in der Aminierung von Phenylpyruvat mit Hilfe einer Transaminase. Obwohl verschiedene Transaminasen in der Lage sind, Phenylpyruvat zu L-Phenylalanin zu transaminieren [Biochem. J. 234,593-604 (1986)], wird diese Funktion in der Zelle hauptsächlich von der sogenannten aromatischen Transaminase erfüllt. Die Abkürzung für das Gen der aromatischen Transaminase in E. coli ist tyrB [Umbarger, Ann. Rev. Biochemistry 47, 533-606 (1978)]. Im Falle von E. coli K12 ist die Lage des Gens auf dem "Bakterienchromosom" genau bekannt. Das Genprodukt hat die E.C.-Nummer 2.6.1.5 erhalten [Bachmann et. al., Microbiological Reviews 44, 1-56 (1980)].

In der Europäischen Patentanmeldung 116 860 wird die Isolierung des tyrB-Gens aus E. coli K12 beschrieben sowie die Klonierung dieses Aminotransferase-Gens auf ein Multi-Copy-Plasmid. Das erhaltene rekombinante Plasmid wird wieder zurück in den Stamm gegeben, aus dem das Gen ursprünglich isoliert worden war, mit dem Ergebnis, daß die L-Phenylalanin-Produktion in diesem Stamm um etwa 11 % erhöht werden konnte.

In der Deutschen Patentanmeldung P 36 31 829.9 ist ein Verfahren vorgeschlagen, nach dem die Isolierung des in E. coli B (ATCC 11303) vorhandenen tyrB-Gens und dessen Klonierung auf ein Multi-Copy-Plasmid nach der Transformation von L-Phenylalanin produzierenden Mikroorganismen mit diesem Plasmid in einer 10-fachen Steigerung der L-Phenylalaninausbeute resultiert.

In dem angeführten Verfahren wurde L-Asparaginsäure als Aminogruppendonor eingesetzt. Anstelle von L-Asparaginsäure können jedoch deren kostengünstigere Vorstufen Fumarsäure und Ammoniumionen eingesetzt werden. Fumarsäure oder Fumarat wird mit Hilfe des in Mikroorganismen vorkommenden Enzyms Aspartase in Gegenwart von Ammoniumionen oder Harnstoff zu L-Asparaginsäure umgesetzt. Nach der Europäischen Patentanmeldung 151 488 werden u.a. mit E. coli ATCC 11303 Ammoniumfumarat und Phenylpyruvat im Verhältnis 2 : 1 mit ca. 80 %iger Ausbeute, bezogen auf Phenylpyruvat, zu L-Phenylalanin umgesetzt. Aspartase kodierende Gene sind bereits kloniert worden [Guest et al., J. Gen. Microbiol. 130, 1271-1278 (1984); Takagi et al., Nucl. Acids Res. 13, 2063-2074 (1985); EP 123 903] und können für die Produktion von L-Asparaginsäure eingesetzt werden (EP 123 903; EP 129 119).

Es hat sich nun gezeigt, daß ein mit einem für aromatische Transaminase kodierenden Plasmid transformierter Mikroorganismus, der gemäß der Deutschen Patentanmeldung P 36 31 829.9 hergestellt werden kann, im Vergleich zu dem genetisch nicht manipulierten E. coli B, keine Erhöhung der L-Phenylalanin-Syntheserate aus den Vorstufen Phenylpyruvat, Fumarsäure und Ammoniumionen oder Harnstoff zeigt. Überraschend wurde jedoch gefunden, daß mit Hilfe von Mikroorganismen, die sowohl ein Aspartase-Gen (aspA), als auch ein für aromatische Transaminase (tyrB) kodierendes Gen kloniert enthalten, demgegenüber eine Steigerung der Syntheserate erzielt werden kann.

Die Erfindung betrifft somit:

1. Verfahren zur Herstellung von L-Phenylalanin mit Hilfe von rekombinanten Bakterien, das dadurch gekennzeichnet ist, daß

a) ein extra-chromosomales Element, das ein aus einem gramnegativen Mikroorganismus isoliertes Aminotransferase und Aspartase kodierendes Gen enthält, oder zwei extra-chromosomale Elemente, die miteinander kompatibel sind, wobei das eine Element ein aus einem gramnegativen Mikroorganismus isoliertes Aminotransferase-Gen und das andere Element ein aus einem gramnegativen Mikroorganismus isoliertes Aspartase-Gen enthält, in einen Mikroorganismus gebracht wird,

b) das Aminotransferase-Gen und das Aspartase-Gen in dem Mikroorganismus exprimiert und eine aktive aromatische Transaminase und Aspartase synthetisiert werden und

c) die Aminotransferase und die Aspartase die Biotransformation von Fumarsäure oder deren Salze und Phenylpyruvat in Gegenwart von Ammoniumionen oder Harnstoff zu L-Phenylalanin bewirken.

2. Ein replizierendes extra-chromosomales Element, das ein aus einem gramnegativen Mikroorganismus isoliertes für Aminotransferase und Aspartase kodierendes Gen enthält.

3. Die Verwendung des unter 2. charakterisierten extrachromosomalen Elements zur Synthese der Aminotransferase und der Aspartase sowie zur Überproduktion von L-Phenylalanin in Mikroorganismen.

Im folgenden wird die Erfindung detailliert beschrieben, insbesondere in ihren bevorzugten Ausführungsformen. Ferner wird die Erfindung in den Patentansprüchen definiert.

Als Quelle für Aspartase und Aminotransferase kodierende Gene können grundsätzlich alle Mikroorganismen verwendet werden, die diese Gene besitzen. Darunter sind nicht nur Mikroorganismen zu verstehen, die beide Gene in ihrem Genom haben, sondern auch solche, die nur eines beider Gene aufweisen, gleichgültig ob dieses konstitutiv oder induzierbar transkribiert wird. Als Mikroorganismen, die beide Gene besitzen, sind beispielsweise die Bakterien der Gattungen Serratia, Escherichia, Pseudomonas, Enterobacter, Salmonella, Erwinia, Proteus, Citrobacter, Paracoccus, Arthrobacter, Bacillus, Brevibacterium, Corynebacterium, Flavorbacterium, Klebsiella, Micrococcus oder Kluyvera zu nennen. Bevorzugt werden die Gene aus gramnegativen Bakterien, insbesondere den Enterobakterien sowie Bakterien der Gattung Pseudomonas, isoliert. Besonders bevorzugt werden die Gene aus E. coli, wie beispielsweise E. coli K12, E. coli B und E. coli W, sowie Serratia marcescens und Pseudomonas fluorescens verwendet. Die Klonierung verschiedener Aminotransferasegene (tyrB, aspC, ilvE) aus E. coli ist nacharbeitbar beschrieben (EP 116 860; DP 36 31 829.9; Fotheringham I.G. et al., Biochem. J. 234, 593-604 (1986)). Die Isolierung von Aspartasegenen aus verschiedenen Bakterienstämmen ist ebenfalls bereits beschrieben, aus E. coli K12 in Guest J.R. et al., J. Gen. Microbiol. 130, 1271-1278 (1984) und in Komatsubara S. et al. J. Biotechnol. 3, 281-291 (1986); aus E. coli W in Takagi J.S. et al., Nucleic Acids Res. 13, 2063-2074 (1985); aus Serratia marcescens in EP 123 903; aus Pseudomonas fluorescens in Takagi J.S. et al., J. Biochem., 100, 697-705 (1986). Bevorzugt wird aspA als Gen für die Aspartase sowie tyrB ilvE und aspC als Gene für die Aminotransferase, mit besonderer Bevorzugung des tyrB-Gens, aus E. coli für die erfindungsgemäße L-Phenylalaninproduktion eingesetzt.

Die Gene können ebenfalls aus vorher genetisch manipulierten Mikroorganismen, bzw. von Plasmiden, die diese Gene enthalten, isoliert werden. Zur Isolierung der aspA-und tyrB-Gene aus Plasmiden werden bevorzugt die bereits beschriebenen bzw. vorgeschlagenen Multi-Copy-Plasmide pGS73 [Guest J.R. et al., J. Gen. Microbiol. 130, 1271-1278 (1984)] und pIMS6056 [Deutsche Patentanmeldung P 36 31 829.9] verwendet. Mit Hilfe von Restriktionsenzymen wird das Aspartase kodierende Gen aspA aus pGS73 isoliert. Das Plasmid wird vorzugsweise mit dem Restriktionsenzym BclI oder einer Kombination der Enzyme SphI und SalI geschnitten, so daß man das Aspartase-Gen auf einem 2,9 kb oder 6,2 kb Fragment erhält. Das tyrB-Gen ist nach Spaltung mit dem Restriktionsenzym SalI oder einer Kombination der Enzyme SalI und SphI aus dem Plasmid pIMS6056 erhältlich. Darüberhinaus sind die aspA-und tyrB-Gene der Plasmide pGS73 und pIMS6056 durch Spaltung mit einer Reihe weiterer Restriktionsendonukleasen zu isolieren [Guest, J.R. et al. J. Gen. Microbiol. 130, 1271-1278 (1984); Deutsche Patentanmeldung P 36 31 829.9.]

Nach der Isolierung beider Gene werden diese auf ein geeignetes Replikon kloniert, beispielsweise das E. coli Multi-Copy-Plasmid pBR322 [Bolivar F. et al., Gene 2, 95-113 (1977)] oder das Plasmid RSF1010 [Guerry P. et al., J. Bacteriol. 117, 619-630 (1974)], das sich gegenüber pBR322 durch ein breiteres Wirtsspektrum und geringere Kopienzahl auszeichnet, sowie deren Derivate. Prophagen-DNS kann ebenfalls als Replikon eingesetzt werden, z.B. das P1-Prophagengenom. Besonders bevorzugt werden zur Konstruktion der erfindungsgemäßen Hybridplasmide in E. coli die Plasmide pGS73 und pIMS6056 verwendet. Die isolierten Gene können dann jeweils auf das Plasmid gebracht werden, auf dem das Gen noch nicht enthalten ist.

Die Ablesung beider Gene erfolgt von zwei verschiedenen Promotoren aus, die den entsprechenden chromosomalen aspA-und tyrB-Genen jeweils vorangeschaltet sind. Alternativ können beide Gene auch gemeinsam oder getrennt mit Hilfe eines starken fremden Promotors, der konstitutiv oder reguliert aktiv ist, transkribiert werden.

Es ist auch möglich, daß das aspA-bzw. tyrB-Gen jeweils in zwei verschiedene kompatible Plasmide kloniert wird, die in der Mikroorganismen-Zelle später nebeneinander bestehen können.

Die Hybridplasmide, die die genannten Gene zusammen oder einzeln kloniert enthalten, werden in kompetente Mikroorganismen-Zellen transformiert. Dazu geeignet sind wieder die vorgehend aufgeführten Mikroorganismen, aus denen die Gene auch isoliert werden können. Vorteilhaft werden die Mikroorganismen transformiert, aus denen vorher die Gene isoliert wurden. Besonders bevorzugt wird das Gen in kompetente E. coli-Zellen eingeschleust. Ampicillinresistente Klone werden durch Restriktionsanalyse auf das Vorhandensein des gewünschten Hybridplasmids untersucht. Aspartase und aromatische Transaminase kodierende Klone können über viele Passagen ohne Verlust der jeweiligen Hybridplasmide zur L-Phenylalaninproduktion aus Phenylpyruvat und Ammoniumfumarat bzw. Fumarsäure oder Fumarat und Ammoniumionen oder Harnstoff eingesetzt werden. Die L-Phenylalaninsyntheserate wird im Vergleich zu genetisch nicht veränderten E. coli Stämmen, bzw. solchen, die lediglich ein kloniertes aspA-oder tyrB-Gen enthalten, um das 2 bis 3-fache erhöht.

Anhand von Beispielen wird die Erfindung weitergehend erläutert. Prozentangaben, sofern nicht anders angegeben, beziehen sich auf das Gewicht.

**Beispiel 1:**

Isolierung von Plasmiden aus E. coli

Das Plasmid pIMS6056 enthält ein aromatische Transaminase kodierendes Gen (tyrB) aus E. coli B (ATCC 11303). pIMS6056 wurde, wie in der Deutschen Patentanmeldung P 36 31 829.9 vorgeschlagen, isoliert. Das Aspartase kodierende Plasmid pGS73 wurde nach Guest J.R. et al., J. Gen. Microbiol. 130, 1271-1278 (1984) gewonnen. pGS73 enthält ein aus E. coli (CS 520) kloniertes aspA-Gen, welches aus der von L. Clarke et al. isolierten E. coli Genbank gewonnen wurde [Clarke L. et al., Cell 9, 91-99 (1976)]. Das Plasmid pIMS6056 wurde aus E. coli B (ATCC 11303), das Plasmid pGS73 aus E. coli K12 (GM242) nach der sog. "cleared lysate"-Methode [Clewell D.B., Helinski D.R., Proc. Natl. Acad. Sci. 62, 1159-1166 (1969)], durch alkalische Lyse [Ish-Horowicz D., Burke J.F., Nucl. Acids Res. 9, 2985-2998 (1981)] oder durch Lyse nach Hitzebehandlung [Holmes D.S. et al., Anal. Biochem. 114, 193 (1981)] präpariert. Die Abtrennung chromosomaler DNS wurde durch Cäsiumchlorid-Ethidiumbromid-Dichtegradientenzentrifugation erreicht (T. Maniatis et al, Molecular Cloning, 1982, S. 93 - 94).

**Beispiel 2:**

Herstellung geeigneter DNS-Fragmente

a) DNS-Fragmente mit überstehenden Enden:

Die aus E. coli isolierten Plasmide pGS73 und pIMS6056 wurden jeweils mit den beiden Restriktionsendonukleasen SalI und SphI verdaut. Dazu wurden jeweils 2 µg Plasmid-DNS eingesetzt und nach den Angaben des Herstellers des Enzyms, Boehringer Mannheim, in einem Gesamtvolumen von 50 µl zunächst mit dem Enzym SalI vollständig gespalten.
Die Reaktion wurde durch Extraktion mit Phenol/Chloroform und Chloroform/Isoamylalkohol beendet. Nach Ethanolfällung und Nachwaschen mit 70 % Ethanol wurde das getrocknete DNS-Pellet in 50 µl des für SphI beschriebenen Inkubationspuffers (Boehringer Mannheim) gelöst und mit dem Enzym SphI vollständig nachgespalten.

b) DNS-Fragmente mit glatten Enden:

Das Plasmid pGS73 wurde mit dem Restriktionsenzym BclI, das Plasmid pIMS6056 mit dem Enzym SalI vollständig gespalten, wobei nach den Angaben des Herstellers der Enzyme, Boehringer Mannheim, verfahren wurde. Die Reaktion wurde wie in Beispiel 2a) beschrieben beendet, die DNS mit Ethanol gefällt und mit 70 % Ethanol gewaschen.
Die überstehenden Enden der entstandenen Plasmid-Fragmente wurden mit DNS-Polymerase I aus E. coli (Klenow Fragment) aufgefüllt. Dazu wurde das getrocknete DNS-Pellet in 18 µl destilliertem Wasser gelöst und mit 2,5 µl Puffer (0,5 M Tris • HCl, pH 7,2, 0,1 M MgSO₄, 1mM Dithiothreit, 500 µg/ml Rinderserumalbumin), 1 µl 2 mM dATP, 1 µl 2 mM dTTP, 1 µl 2 mM dCTP, 1 µl 2 mM dGTP und 2 Einheiten DNS-Polymerase I (Klenow-Fragment) 30 Minuten bei 22°C inkubiert. Die Reaktion wurde durch Erhitzen über 5 Minuten auf 70°C beendet.

**Beispiel 3:**

Behandlung von Plasmid-DNS mit alkalischer Phosphatase

Die endständigen Phosphatgruppen der nach Beispiel 2a oder 2b hergestellten Fragmente des Plasmids pIMS6056 wurden jeweils entfernt durch Inkubation über 30 Minuten bei 37°C mit 23 Einheiten alkalischer Phosphatase aus Kälberdarm. Der Ansatz wurde mit Phenol/Chloroform und Chloroform/Isoamylalkohol extrahiert, mittels Ethanol gefällt und mit 70 % Ethanol gewaschen. Nach dem Trocknen unter Vakuum wurde die DNS in 20 µl TE-Puffer (10 mM Tris• HCl, 1 mM EDTA, pH 8,0) gelöst.

**Beispiel 4:**

Isolierung von DNS-Fragmenten

Die nach Beispiel 2a oder 2b erhaltenen DNS-Fragmente des Plasmids pGS73 wurden jeweils elektrophoretisch aufgetrennt in einem Gel aus 1 % niederschmelzender ("low-melting-temperature") Agarose mit TAE [40 mM Tris, 10 mM Natriumacetat, 1 mM EDTA (pH 7,8)] als Laufpuffer. Das 6,2 kb SalI/SphI-Fragment (Beispiel 2a) bzw. das 2,9 kb Fragment mit glatten Enden (Beispiel 2b) wurde nach Anfärben des Gels mit Ethidiumbromid und Bestrahlung mit langwelligem UV-Licht (366 nm) jeweils als Bande sichtbar gemacht, mit Hilfe eines DNS-Längenstandards (Boehringer Mannheim, Katalog Nr. 236250) identifiziert, herausgeschnitten, 5 Minuten bei 65°C inkubiert, zuerst mit Tris-gesättigtem Phenol pH 8,0 und anschließend mit Phenol/Chloroform und Chloroform/Isoamylalkohol extrahiert. Aus der wäßrigen Phase wurde die DNS jeweils mit Ethanol gefällt, mit 70 % Ethanol gewaschen und nach dem Trocknen unter Vakuum mit 20 µl TE-Puffer resuspendiert.

**Beispiel 5:**

Ligierung von DNS-Fragmenten

a) Ligierung überstehender Enden

Die nach Beispiel 3 erhaltenen dephosphorylierten Sall/SphI-Fragmente des Plasmids pIMS6056 wurden mit dem gemäß Beispiel 4 isolierten 6,2 kb Sall/SphI-Fragment des Plasmids pGS73 in einem molaren Verhältnis von etwa 3:1 gemischt und in dem vom Hersteller, Boehringer Mannheim, empfohlenen Inkubationspuffer mit 1 Einheit T4-DNS-Ligase bei 16°C über 16 Stunden inkubiert. Das Gesamtvolumen des Ansatzes betrug dabei 50 µl mit einer Gesamt-DNS-Konzentration von 20 µg/ml.

b) Ligierung glatter Enden

Das nach Beispiel 2b gewonnene 8,2 kb pIMS6056-Fragment wurde nach Dephosphorylierung nach Beispiel 3 für die Ligase-Reaktion eingesetzt. Dazu wurde das pIMS6056-Fragment mit dem nach Beispiel 4 isolierten 2,9 kb Fragment des Plasmids pGS73 in einem Verhältnis von etwa 3:1 gemischt und mit 50 mM Tris • Cl, pH 7,6, 10 mM MgCl$_2$, 5 % (w/v) Polyethylenglykol 8.000, 1 mM ATP, 1 mM Dithiothreit, 1 Einheit T4-DNS-Ligase bei 25°C über 4 Stunden inkubiert. Das Gesamtvolumen des Ansatzes betrug 20 µl mit einer Gesamt-DNS-Konzentration von 10 µg/ml.

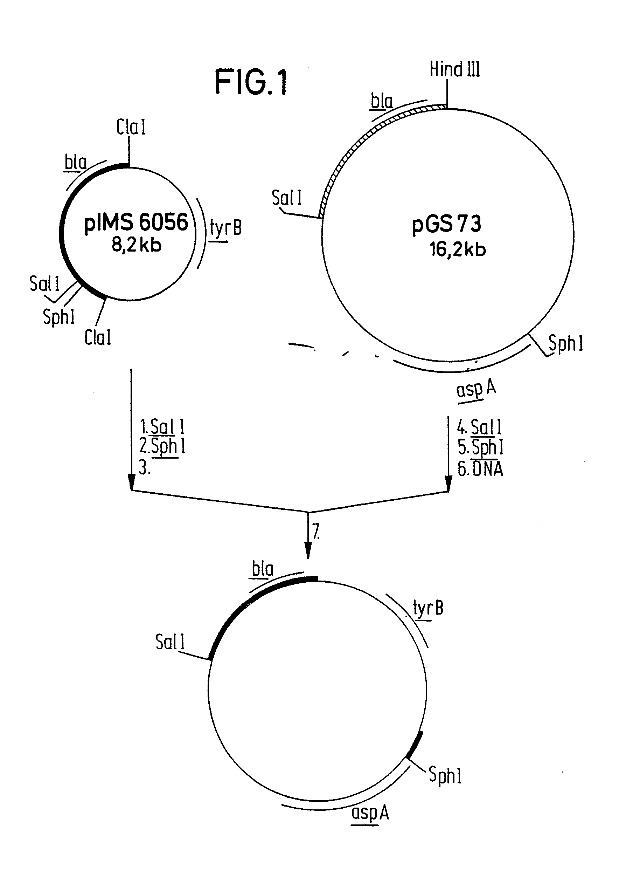

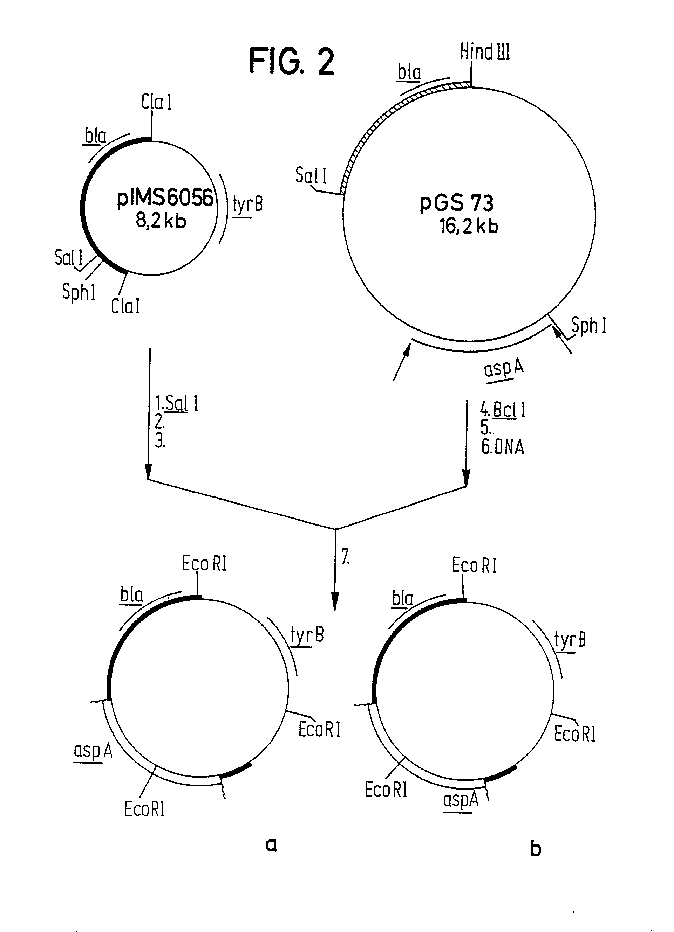

Die Konstruktion der Plasmide ist in den Figuren 1 und 2 dargestellt. Figur 1 zeigt die Konstruktion eines aromatische Transaminase (tyrB) und Aspartase (aspA) kodierenden Plasmids: Klonierung überstehender Enden. Figur 2 zeigt die entsprechende Klonierung glatter Enden. Folgende Abkürzungen werden verwendet: bla = Ampicillin-Resistenzgen (β-Lactamasegen); der ausgefüllte Balken entspricht pAT153-DNA, der gestrichelte Balken pBR322-DNA. Die beiden Pfeile in Figur 2 geben die Position des 2,9 kb Bcl I Fragments auf dem Plasmid pGS73 an.

**Beispiel 6:**

Transformation von E. coli K12 Stämmen

Der nach Beispiel 5a) oder 5b) hergestellte Ligationsansatz wurde 3-fach verdünnt und für die Transformation von E. coli K12 (beispielsweise HB101 oder W3110) eingesetzt nach der in T. Maniatis et al. beschriebenen Methode (T. Maniatis et al., Molecular Cloning, 1982, CSHL, New York, S. 250 - 251). Der Transformationsansatz wurde ausplattiert auf Luria-Bertani Medium (1 % Trypton, 0,5 % Hefeextrakt, 1 % Natriumchlorid, pH 7,5, 1,8 % Agar), welches nach dem Autoklavieren mit 100 µg/ml Ampicillin versetzt wurde.

**Beispiel 7:**

Identifizierung der Aspartase und aromatische Transaminase kodierenden Klone

Für die Isolierung von Transformanden, die das gewünschte Plasmid enthielten, wurde die Plasmid-DNS von Ampicillin-resistenten Klonen isoliert [Ish-Horowicz D., Burke J.F., Nucl. Acids Res. 13, 2989-2998 (1981)] und nach vollständiger Verdauung mit den Restriktionsenzymen Sall und SphI (Beispiel 5a) bzw. EcoRI (Beispiel 5b) in einem 0,8 %igen Agarosegel elektrophoretisch aufgetrennt. Rekombinante Plasmide des Ligationsansatzes nach Beispiel 5a, die das 6,2 kb Sall/SphI-Fragment aus pGS73 aufwiesen neben dem 8,1 kb Sall/SphI-Fragment des Vektorplasmids pIMS6056, enthielten jeweils ein kloniertes aspA-und tyrB-Gen (s. Abb. 1). Diese Hybridplasmide wurden in E. coli auch in Abwesenheit von Ampicillin stabil erhalten.

Rekombinante Plasmide des Ligationsansatzes nach Beispiel 5b, welche drei Schnittstellen für das Enzym EcoRI aufwiesen, enthielten ebenfalls jeweils ein kloniertes Gen für Aspartase und aromatische Transaminase (s. Abb. 2). Durch Größenvergleich der entstandenen Fragmente ergab sich, daß das Aspartase kodierende 2,9 kb DNS-Fragment aus pGS73 in beiden Orientierungen in das Plasmid pIMS6056 kloniert worden was (s.

Abb. 2). Die beiden auf diese Weise isolierten Plasmide enthielten das 2,9 kb Fragment aus pGS73 in unterschiedlicher Orientierung relativ zur pIMS6056 DNS, und wurden in E. coli auch in Abwesenheit von Ampicillin stabil erhalten.

**Beispiel 8:**

Transformation von E. coli B Stämmen

Die gemäß Beispiel 7 isolierten Aspartase und aromatische Transaminase kodierenden Plasmide wurden aus E. coli K12 (beispielsweise HB101 oder W3110) gemäß Beispiel 1 isoliert und für die Transformation von E. coli B ATCC 11303 eingesetzt nach der in Beispiel 6 beschriebenen Methode. Die Ausbeute Ampicillin resistenter Klone war jedoch im Vergleich zu E. coli K12 Stämmen sehr niedrig. Die Plasmide Ampicillin-resistenter Transformanden von E. coli B ATCC 11303 wurden isoliert und durch Restriktionsanalyse gemäß Beispiel 7 erneut untersucht.

**Beispiel 9:**

Bestimmung der Aspartase-und aromatische Transaminase-Aktivität

Aspartase-Aktivität wurde mit dem Sigma Test-Kit A-8147 bestimmt. Die Messung der aromatischen Transaminase-Aktivität erfolgte mit den Sigma Test-Kit G0390, wobei statt $\alpha$-Ketoglutarat 12 mmol/l Phenylpyruvat (Na-Salz) eingesetzt wurde. Extrakte der gemäß Beispiel 7 oder 8 erhaltenen rekombinanten E. coli K 12-oder B-Stämme, welche sowohl ein aspA-Gen als auch ein tyrB-Gen auf einem Plasmid kloniert enthielten, wiesen im Vergleich zu dem entsprechenden plasmidfreien Stamm ca. 5-10-fach erhöhte Aspartase-und aromatische Transaminase-Aktivität auf.

**Beispiel 10:**

Verfahren zur L-Phenylalaninherstellung aus Phenylpyruvat, Fumarsäure und Ammoniumionen

E. coli B (ATCC 11303) wurde 20 Stunden in 10 l folgender Nährlösung bei 37°C kultiviert:

| | |
|---|---|
| Fumarsäure | 30 g/l |
| Fleischextrakt | 20 g/l |
| $KH_2PO_4$ | 2 g/l |
| $MgSO_4 \bullet 7 H_2O$ | 0,5 g/l |
| $CaCl_2 \bullet 2 H_2O$ | 0,1 g/l |
| $NH_4Cl$ | 1 g/l. |

pH 7,9 wurde mit Ammoniak eingestellt. 24 g/l Phenylpyruvat wurden sterilfiltriert zugegeben.

Das erhaltene Zellmaterial wurde in 100 ml einer wäßrigen Lösung aus 30 g/l Phenylpyruvat Natriumsalz, 30 g/l Fumarsäure, welche mit Ammoniak neutralisiert wurde, 30 g/l Ammoniumchlorid, 0,6 g/l $MgCl_2 \bullet 6H_2O$, 0,1 % Polyoxyethylensorbitanmonooleat (®Tween 80), und 50 mM Tris $\bullet$ HCl (pH 7,9) suspendiert. Die Suspension wurde bei 37°C unter Begasung mit 0,5 VVm Preßluft inkubiert. Nach 9 Stunden konnte eine L-Phenylalaninkonzentration von 23 g/l gemessen werden.

**Beispiel 11:**

Einsatz der isolierten rekombinanten Bakterien bei der L-Phenylalaninherstellung

E. coli Stämme, welche eines der in Beispiel 7 beschriebenen Aspartase und aromatische Transaminase kodierenden Plasmide enthielten, wurden für die L-Phenylalaninproduktion aus Phenylpyruvat nach der in Beispiel 10 beschriebenen Methode eingesetzt. Bereits nach 3 bis 5 Stunden konnte eine L-Phenylalaninkonzentration von 24 g/l bestimmt werden.

**Ansprüche**

1. Verfahren zur Herstellung von L-Phenylalanin mit Hilfe von rekombinanten Bakterien dadurch gekennzeichnet, daß

a) ein extra-chromosomales Element, das ein aus einem gramnegativen Mikroorganismus isoliertes Aminotransferase und Aspartase kodierendes Gen enthält, oder

zwei extra-chromosomale Elemente, die miteinander kompatibel sind, wobei das eine Element ein aus einem gramnegativen Mikroorganismus isoliertes Aminotransferase-Gen und das andere Element ein aus einem gramnegativen Mikroorganismus isoliertes Aspartase-Gen enthält, in einen Mikroorganismus gebracht wird,

b) das Aminotransferase-Gen und das Aspartase-Gen in dem Mikroorganismus exprimiert und eine aktive Aminotransferase und Aspartase synthetisiert werden und

c) die Aminotransferase und die Aspartase die Biotransformation von Fumarsäure oder deren Salze und Phenylpyruvat in Gegenwart von Ammoniumionen oder Harnstoff zu L-Phenylalanin bewirken.

2. Das Verfahren nach Anspruch 1, dadurch gekennzeichnet, daß der Mikroorganismus, in dem die Gene exprimiert werden, ein Enterobakterium oder ein Bakterium der Gattung Pseudomonas ist.

3. Das Verfahren nach Anspruch 2, dadurch gekennzeichnet, daß der Mikroorganismus ein E. coli K12-Stamm, ein E. coli B-Stamm oder ein E. coli W-Stamm ist.

4. Ein replizierendes extra-chromosomales Element, das ein aus einem gramnegativen Mikroorganismus isoliertes Aminotransferase und Aspartase kodierendes Gen enthält.

5. Das extra-chromosomale Element nach Anspruch 4, dadurch gekennzeichnet, daß es die Gene aus Enterobakterien und/oder aus Bakterien der Gattung Pseudomonas enthält.

6. Das extra-chromosomale Element nach Anspruch 5, dadurch gekennzeichnet, daß es die Gene aus E. coli K12-Stämmen und/oder E. coli B-Stämmen und/oder E. coli W-Stämmen sowie Serratia marcescens und/oder Pseudomonas fluorescens enthält.

7. Das extra-chromosomale Element nach Anspruch 6, dadurch gekennzeichnet, daß es das aspA-Gen aus E. coli K12 und das tyrB-Gen aus E. coli ATCC 11303 enthält.

8. Das extra-chromosomale Element nach einem oder mehreren der Ansprüche 5 bis 7, dadurch gekennzeichnet, daß es ein Multi-Copy-Plasmid ist.

9. Die Verwendung des extra-chromosomalen Elements nach einem oder mehreren der Ansprüche 5 bis 8 zur Synthese der Aminotransferase und der Aspartase.

10. Die Verwendung des extra-chromosomalen Elements nach einem oder mehreren der Ansprüche 5 bis 8 zur Überproduktion von L-Phenylalanin in Mikroorganismen.

Patentansprüche für folgende Vertragsstaaten : ES, GR.

1. Verfahren zur Herstellung von L-Phenylalanin mit Hilfe von rekombinanten Bakterien dadurch gekennzeichnet, daß

a) ein extra-chromosomales Element, das ein aus einem gramnegativen Mikroorganismus isoliertes Aminotransferase und Aspartase kodierendes Gen enthält, oder

zwei extra-chromosomale Elemente, die miteinander kompatibel sind, wobei das eine Element ein aus einem gramnegativen Mikroorganismus isoliertes Aminotransferase-Gen und das andere Element ein aus einem gramnegativen Mikroorganismus isoliertes Aspartase-Gen enthält, in einen Mikroorganismus gebracht wird,

b) das Aminotransferase-Gen und das Aspartase-Gen in dem Mikroorganismus exprimiert und eine aktive Aminotransferase und Aspartase synthetisiert werden und

c) die Aminotransferase und die Aspartase die Biotransformation von Fumarsäure oder deren Salze und Phenylpyruvat in Gegenwart von Ammoniumionen oder Harnstoff zu L-Phenylalanin bewirken.

2. Das Verfahren nach Anspruch 1, dadurch gekennzeichnet, daß der Mikroorganismus, in dem die Gene exprimiert werden, ein Enterobakterium oder ein Bakterium der Gattung Pseudomonas ist.

3. Das Verfahren nach Anspruch 2, dadurch gekennzeichnet, daß der Mikroorganismus ein E. coli K12-Stamm, ein E. coli B-Stamm oder ein E. coli W-Stamm ist.

4. Verfahren zur Herstellung eines replizierenden extra-chromosomalen Elements zur Expression einer aktiven Aminotransferase und Aspartase in einem Bakterium, dadurch gekennzeichnet, daß ein aus einem gramnegativen Mikroorganismus isoliertes, Aminotransferase und Aspartase kodierendes, Gen in ein Plasmid kloniert wird.

5. Verfahren nach Anspruch 4, dadurch gekennzeichnet, daß die Gene aus Enterobakterien und/oder aus Bakterien der Gattung Pseudomonas verwendet werden.

6. Verfahren nach Anspruch 5, dadurch gekennzeichnet, daß die Gene aus E. coli K12-Stämmen und/oder E. coli B-Stämmen und/oder E. coli W-Stämmen sowie Serratia marcescens und/oder Pseudomonas fluorescens verwendet werden.

7. Verfahren nach Anspruch 6, dadurch gekennzeichnet, daß das aspA-Gen aus E. coli K12 und das tyrB-Gen aus E. coli ATCC 11303 verwendet werden.

8. Verfahren nach einem oder mehreren der Ansprüche 4 bis 7, dadurch gekennzeichnet, daß als Plasmid ein Multi-Copy-Plasmid verwendet wird.

FIG.1

# FIG. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A1 - 0 116 860 (G.D. SEARLE & CO.)  \* Ansprüche 1-3,5-7,9-15, 17-19 \* | 1,2,4, 5,9,10 | C 12 N 15/00 <br> C 12 P 13/22 <br> C 12 N  9/10 <br> //(C 12 N 15/00; <br> C 12 R  1:38) <br> (C 12 N 15/00; <br> C 12 R  1:19) |
| D,A | EP - A2 - 0 151 488 (KYOWA HAKKO KOGYO CO., LTD.)  \* Anspruch 1 \* | 1,2 | (C 12 N 15/00; <br> C 12 R  1:43) <br> (C 12 N 15/00; <br> C 12 R  1:39) |
| A | EP - A1 - 0 137 646 (GENENTECH, INC.)  \* Ansprüche 1-4 \* | 1,2 | |
| D,A | EP - A2 - 0 129 119 (TANABE SEIYAKU CO., LTD.)  \* Zusammenfassung \* | 1,6 | |
| D,A | EP - A2 - 0 123 903 (TANABE SEIYAKU CO., LTD.)  \* Zusammenfassung \* | 1,6 | RECHERCHIERTE SACHGEBIETE (Int. Cl.4) <br><br> C 12 N <br> C 12 P |
| D,A | ANNUAL REVIEW OF BIOCHEMISTRY, Band 47, 1978, Palo Alto, Kalifornien, USA  <br> H.E. UMBARGER "Amino Acid Biosynthesis and its Regulation" Seiten 533-606  \* Seiten 581, 582 \* | | |

Der vorliegende Recherchenbericht wurde für alle Patentansprüche erstellt.

| Recherchenort | Abschlußdatum der Recherche | Prüfer |
|---|---|---|
| WIEN | 26-07-1988 | WOLF |